# EUROPEAN PATENT APPLICATION

(11) **EP 1 795 589 A1**
(43) Date of publication of application: **13.06.2007**
(21) Application number: 06125495.9
(22) Date of filing: 06.12.2006
(51) Int. Cl.: C12N 5/06

(54) **Method for the direct culture of dendritic cells without a preceding centrifugation step**

(30) Priority: 07.12.2005 EP 05111806
(71) Applicant: Schuler, Gerold, Prof. Dr., 91080 Spardorf (AT)
(72) Inventor: Schuler, Gerold, Prof. Dr., 91080 Spardorf (DE); Schuler-Thurner, Beatrice, 91080 Spardorf (DE); Erdmann, Michael Karl, 91080 Spardorf (DE)
(74) Representative: von Kreisler Selting Werner

(57) **Abstract**

The present invention provides a method for the direct culture of dendritic cell precursors enriched by elutriation without a preceding centrifugation step to generate dendritic cells.

## Description

The present invention provides a method for the direct culture of dendritic cell precursors enriched by elutriation without a preceding centrifugation step to generate dendritic cells.

### Background of the Invention

The adoptive transfer of antigen-loaded dendritic cells (DC), i.e. DC vaccination, is a promising approach to manipulate the immune system (reviewed in Banchereau J. and Palucka A. K., Nat. Rev. Immunol. 5(4):296-306 (2005) and Nestle F. O. et al., Curr. Opin. Immunol. 17(2): 163-9 (2005). In murine models mature DC ("immunostimulatory DC") have been used to induce immunity to cancer and infection by inducing effector T cells, and immature or semimature DC ("tolerogenic DC") were employed to induce antigen-specific tolerance by inducing regulatory T cells. In man more than 1000 cancer patients have been vaccinated with "immunostimulatory DC" in clinical trials. DC vaccination appeared particularly immunogenic, and in some clinical trials promising clinical effects have been observed. DC vaccination is, however still in an early stage of development. Many critical variables have not yet been addressed (e.g. DC type and maturation stimulus; dose, frequency, and route of injection etc.). A major problem is the fact that the generation of DC vaccines is still expensive and time consuming and at the same time not yet standardized, in part because a straightforward closed system is not yet available.

DC are most often generated from monocytes. To this end adherent fractions of peripheral blood mononuclear cells (isolated from freshly drawn blood or aphereses which are highly enriched in monocytes (Putz T. et al., Methods Mol. Med. 109:71-82 (2005)), or monocytes isolated from peripheral blood, e.g. by magnetic separation from apheresis products using the CliniMacs System (Berger T. G. et al., J. Immunol. 268(2):131-40 (2002); Campbell J. D. et al., Methods Mol. Med. 109:55-70 (2005); Babatz J. et al., J. Hematother. Stem Cell Res. 12(5):515-23 (2003) and Motta M. R. et al., Br. J. Haematol. 121(2):240-50 (2003))) are exposed to GM-CSF + IL-4 usually over about 6 days to yield immature DC, and finally matured by adding a maturation stimulus (most often IL-1 alpha + IL-6 + TNF alpha + PGE2). Instead of GM-CSF + IL-4 other cytokine combinations can be used such as GM-CSF + IL13, GM-CSF + IL-15, GM-CSF + IFN alpha etc. (reviewed in Banchereau J. and Palucka A. K., Nat. Rev. Immunol. 5(4):296-306 (2005)), and monocytes can even be converted to DC by the use of CpG oligonucleotides (Krutzik S. R., Nat. Med. 11(6):653-60 (2005)). Also, a variety of maturation stimuli are available (Gautier G. et al., J. Exp. Med. 2;201(9):1435-46 (2005); Napolitani G. et al., Nat. Immunol. 6(8):769-76. Epub 2005 Jul 3 (2005) and reviewed in Banchereau J. and Palucka A. K., Nat. Rev. Immunol. 5(4):296-306 (2005)).

To test these and other variables and to perform larger, randomized clinical trials it is of utmost importance to develop a cost-effective, reproducible method to generate DC in a closed system. Adherence-based closed systems (such as the AastromReplicell System for production of dendritic cells, Aastrom Biosciences, Inc., Ann Arbor, MI, USA) require a large volume of culture media causing high costs of production, and require as an additional preceding step the isolation of PBMC from apheresis products, for which no straightforward, clinically approved system is available. The alternative approach is to first isolate monocytes. This has been possible for some time by using superparamagnetic iron-dextran particles directly conjugated to CD14 antibody and the CliniMACS Cell Selection System (Miltenyi Biotec GmbH, Bergisch Gladbach, Germany, see Berger T. G. et al., J. Immunol. 268(2):131-40 (2002); Campbell J. D. et al., Methods Mol. Med. 109:55-70 (2005); Babatz J. et al., J. Hematother. Stem Cell Res. 12(5):515-23 (2003) and Motta M. R. et al., Br. J. Haematol. 121(2):240-50 (2003)) which is approved for clinical use in Europe but not in the USA. The system yields essentially pure fractions of positively selected monocytes which are delivered from the magnetic columns into bags in CliniMACS PBS/EDTA buffer. Highly enriched fractions of monocytes can also be obtained by immunomagnetic depletion of contaminating of T, B and NK cells (Berger T. G. et al., J. Immunol. Methods 298(1-2):61-72 (2005)) using the Isolex 300I Magnetic cell selector.

We recently described an alternative, and in our view preferable approach to isolate monocytes for DC generation, namely elutriation employing an advice which became recently available and is approved for clinical use (Elutra Cell Separation System^{™}, Gambro AB, Stockholm, Sweden; see Berger T. G. et al., J. Immunol. Methods 298(1-2):61-72 (2005)). Obvious advantages are that the enriched monocytes are untouched, and that the use of costly and non-humanized antibodies for magnetic separation is avoided. A disadvantage shared by the magnetic separation and the elutriation procedures is the fact that the cell factions are obtained in a salt solution, which necessitates at least one concentration step, i.e. a centrifugation step, before the start of the cell culture as the monocytes have to be sedimented for removal of the salt solution and have to be suspended in a suitable culture medium. According to today's standards such centrifugation cannot be performed under sterile conditions, rather has to be performed in an open system, i.e. by opening the hitherto closed container/bag and taking the cell fractions out of the container/bag. Even in a GMP laboratory such taking out is critical. A proposed solution to this problem is to centrifuge the closed container/bag and to remove the supernatant by suction, which is, however, quite cumbersome. This problem does not only occur in case of isolating and cultivating monocytes, but is immanent whenever cells (e.g. lymphocytes), which were isolated either by magnetic separation or the elutriation process, have to be put in culture.

### Summary of the Invention

We found that the elutriation procedure magnetic or other separation strategies can be modified so as to avoid such a potentially non-sterile centrifugation step. The method established allows the *direct* culture of cells enriched by elutriation, magnetic or other separation strategies *without* preceding centrifugation step by performing the enrichment step in a medium close to or identical with the final culture medium.

Thus the present invention provides a method for the sterile separation and culture of blood-derived cells or bone marrow-derived cells, which method comprises enriching the cells by a sterile separation procedure and performing the enrichment or elution step of the separation procedure with an eluation medium close to or identical with the medium required for culture of the cells, and collecting the cells in a vessel under sterile conditions.

The method may comprise subsequent culturing of the cells isolated as described herein before. "Sterile separation and culture" and "sterile separation procedure" according to the invention means that contact with ambient air, such as in non-sterile centrifugation steps are avoided. In particular it is preferred that between the separation procedure and the culturing of the cells no additional centrifugation step is required.

The method further relates to cells cultured by the method defined above.

### Brief Description of the Figures

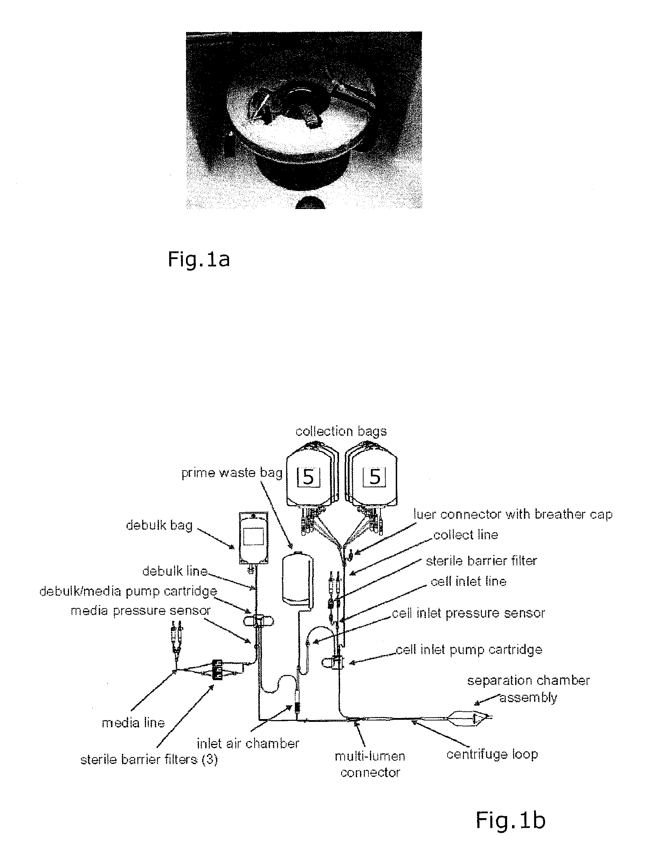

Fig. 1A: Elutra-cell separator: rotor containing the 40-ml elutriation chamber. B: Components of the disposable tubing set. The centrifuge loop consists of the following components: The multi-lumen tubing carries fluid in and out of the separation chamber assembly. The sleeves reinforce the tubing at the flex points. The collars connect the two ends of the loop in the centrifuge. The bearings provide contact points between the centrifuge arm and the loop. The debulk line carries RBCs and some granulocytes from the separation chamber to the debulk bag during RBC debulking. It also carries media solution during the Prime mode and debulk rinse. The debulk/media pump cartridge holds and organizes the debulk and media line tubing. The debulk bag (600 ml) stores red blood cells and some granulocytes removed during RBC debulking. It also stores media solution processed during Prime mode and debulk rinse. The collect line: during Prime mode, the collect line carries the media solution used during Prime mode to the prime collection bag. During Run mode, the collect line carries the collected components to a fraction collection bag. The collect line clamps open and close the lines to the prime collection bag, fraction collection bags, and the line with the blue breather cap. The collection bags (1 I) are manufactured from citrated PVC. The prime collection bag stores the media solution used during Prime mode. The fraction collection bags (5) store the fractions collected. The product sampler provides a means for taking a sample of the fraction collection bags. The prime waste bag stores the media solution used during Prime mode. The waste divert valve lines carry media solution to the prime waste bag during Prime mode.
Fig. 2: Mixed lymphocyte culture. NAF: non adherent fraction during adhesion step, cpm: counts per minute. Allogenic-MLR was performed as described (Thurner et al., 1999). Briefly, 2 x 10⁵ allogeneic non-adherent PBMC per well and DC (1:6.6; 1:20; 1:66; 1:200; 1:666; 1:2000) were co-cultured in triplicates in 96-well flat bottom plates for four days in RPMI 1640 supplemented with gentamicin, glutamine and 5 % heat-inactivated human pool serum. Proliferation of each PBMC/DC-co-cultivation was determined by ³H-Thymidine-assay (4 µCi/ml final concentration added 12-16 h before harvest).
Fig. 3: DC-yield (%) of different leukaphereses of the same patients (standard protocol: pale grey versus elutriation protocol: dark grey). A comparison between different leukaphereses performed of the same patient (n = 14) showed a significant higher DC-yield in the elutriation procedure compared to the standard gradient/plastic adhesion protocol. Higher DC yields resulted in significantly more vaccinations that could be produced per leukapheresis.
Fig. 4: DC-yield of two patient leukaphereses processed partially by standard (pale grey) versus elutriation (dark-grey) protocol. Two patient leukaphereses were separated into two fractions with one part being cultivated with the standard procedure (gradient/plastic adherence) and one part processed using elutriation and cultivation in culture bags. Within the same leukapheresis a higher DC-yield was achieved with elutriation compared to standard protocol.

### Detailed Description of the Invention

The present invention provides for an improved *ex-vivo* process for the culture of blood-derived cells under all-sterile conditions, namely a process meeting the GMP requirements. Cells to be cultured by the method of the invention include dendritic cells (DCs) and all other cells present in or derived from peripheral blood (or bone marrow) including stem cells and their progeny.

In the method described above, the separation process includes, but is not limited to, elutriation and magnetic separation.

In a preferred embodiment an elutriation device, preferably the Elutra^{™} Cell Separation System (Gambro AB, Stockholm, Sweden) Berger T. G. et al., J. Immunol. Methods 298(1-2):61-72 (2005)) is utilized in the separation procedure. Further suitable elutriation devices are the Amicus Separator (Baxter Healthcare Corporation, Round Lake, IL, USA), the Trima Accel® Collection System (Gambro BCT, Lakewood, CO, USA) and the elutriation rotors JE-5.0 and JE-6B (Beckmann Coulter, Inc., Fullerton, CA, USA).

As abundant erythrocytes interfere with effectiveness in elutriation, elutriation devices such as the Elutra^{™} Cell Separation System offer a debulking step to reduce the erythrocyte (RBC: red blood cell) concentration of the leukapheresis product before elutriation. This procedure results in a significant monocyte loss of the leukapheresis product.

In one embodiment of the invention an elutriation device is utilized in the separation procedure of the method of the invention, wherein the elutriation device is operated so to avoid debulking of erythrocytes during elutriation in order to prevent monocyte loss of the leukapheresis product, preferably wherein debulking of erythrocytes is prevented by limiting the input volume of the leukapheresis product to the elutriation device relative to the erythrocyte concentration, more preferably wherein debulking is prevented by limiting the input volume of leukapheresis product to the elutriation device so that the packed erythrocyte volume is less than 20% of the volume of the reaction chamber of the elutriation device (in the preferred Elutra^{™} Cell Separation System having a reaction chamber volume of 40 ml the maximal input volume for packed erythrocytes is 7.5 ml).

Magnetic separation is suitable e.g. for the isolation of monocytes from peripheral blood, and includes their magnetic separation from apheresis products using the CliniMacs System (Berger T. G. et al., J. Immunol. 268(2):131-40 (2002); Campbell J. D. et al., Methods Mol. Med. 109:55-70 (2005); Babatz J. et al., J. Hematother. Stem Cell Res. 12(5):515-23 (2003) and Motta M. R. et al., Br. J. Haematol. 121(2):240-50 (2003)).

Both methods yield pure fractions of the desired cells such as monocytes, which are delivered into closed containers/bags.

In the method of the invention the blood-derived cells or bone marrow-derived cells are preferably selected from monocytes, immature dendritic cells, CD34⁺ cells, stem cells, and any other cell contained therein.

Moreover it is preferred in the method of the invention that the vessel allows for sterile collection and culture of the cell suspension, sterile transfer to other culture or storage vessels, and optionally for sterile cryopreservation. It is preferred that the vessel is a plastic bag having sealable adapters, such as the Cell-Freeze bag type CF100-C3 containing a pre-attached DMSO filter, which allows for closed system cryopreservation.

The method of the invention may further comprise culture, differentiation and/or expansion of the cells under reaction conditions and by addition of supplements required for the culture, differentiation and/or expansion of the respective cells or precursor cells.

In a particular preferred embodiment of the invention, the method is suitable for the sterile culture of dendritic cell precursors to generate dendritic cells and comprises elutriation of peripheral blood mononuclear cells and culture of the monocytes obtained by the elutriation. The invention is hereinafter described by reference to said preferred embodiment, the generation of dendritic cells, which shall, however, not be construed to limit the invention.

In the method of the invention and in particular in a method for the generation of dendritic cells the preferred separation process is elutriation. The buffer for elutriation, i.e. the culture medium suitable for the elutriation and culture, includes, but is not limited to, RPMI medium supplemented with autologous plasma or serum or allogenic plasma or serum, preferably with about 1% of said plasma or serum (such as the commercially available RPMI 1640 medium BE12-167F of Bio Whittaker, Walkersville, USA, supplemented with 20 µg/ml gentamicin (Refobacin 10, Merck, Darmstadt, Germany), 2 mM glutamine (Bio Whittaker), and 1% heat-inactivated (56 °C for 30 min) autologous human plasma), X-Vivo 15 medium (Cambrex Bio Science, Verviers, Belgium), CellGro DC Medium (Cell Genix GmbH Freiburg, Germany), AimV Culture Medium (Invitrogen, Carlsbad, CA, USA), Panserin^{™} 416 (Pan-Biotech GmbH, Aidenbach, Germany), and the like. Particularly preferred is the above-mentioned RPMI medium supplemented with 1% autologous human plasma or serum.

For the generation of dendritic cells the isolated monocytes are cultured while adding one or more differentiation factors such as cytokines and/or one or more maturation factors.

The differentiation factors include, but are not limited to, GM-CSF, IL-4, IL-13, IFN-α, IL-3, IFN-β, TNF-α, CPG oligonucleotides and combinations of said differentiation factors.

In particular, monocytes can be differentiated to stimulatory dendritic cells by the following differentiated factors and combinations of differentiated factors: GM-CSF and IL-4 (Romani, N. et al., J. Exp. Med. 180:83-93 (1994); Sallusto, F. and Lanzavecchia, A., J. Exp. Med. 179:1109-1118 (1994)), GM-CSF and IL-13 (Romani, N. et al., J. Immunol. Methods 196(2):137-51 (1996)) GM-CSF + IFN-α (Carbonneil, C. et al., AIDS 17(12):1731-40 (2003)), IL-3 and IL-4 (Ebner, S. et al., J. Immunol. 168(12):6199-207 (2002)), IL-3 and IFN-β (Buelens, C. et al., Blood 99(3):993-8 (2002)), IFN-α (Blanco, P. et al., Science 294:1540-1543 (2001); Luft, T. et al. J Immunol 161:1947-1953 (1998); Paquette, R. L. et al., J. Leukoc. Biol. 64:358-367 (1998); Santini, S. M. et al., J. Exp. Med. 191:1777-1788 (2000)). TNF-α (Chomarat, P. et al., J Immunol 171:2262-2269 (2003)), GM-CSF and IL-15 (Mohamadzadeh M. et al., J. Exp. Med. Oct 1;194(7):1013-20 (2001)) , CpG oligonucleotides (primarily via induction of GM-CSF and other cytokines; Krutzik S.R. et al., Nat. Med. 11(6):653-60. (2005)). Particular preferred for the differentiation of stimulatory dendritic cells are a combination of GM-CSF and IL-4.

The present method is also applicable for the Generation of tolerogenic dendritic cells. Suitable conditions for the induction of such tolerogenic dendritic cells is e.g. described in the following articles the content of which is hereby incorporated in its entirety: Lutz M. B. and Schuler G., Trends Immunol. 23(9):445-9 (2002); Steinbrink K. et al., Blood 93(5):1634-42 (1999); Rea D. et al., Hum. Immunol. 65(11):1344-55 (2004); Bellinghausen I. et al., J. Allergy Clin. Immunol. 108(2):242-9 (2001); Jonuleit H. et al., J. Exp. Med. 192(9):1213-22 (2000); Dhodapkar M.V. and Steinman R.M., Blood 100(1):174-7 (2002); Levings M. K. et al., Blood 105(3):1162-9 (2002); Rea D. et al., Blood 95(10):3162-7 (2000); and de Jong E. C. et al., J. Leukoc. Biol. 66(2):201-4. (1999).A variety of maturation factors are available. Particular maturation factors and combination of maturation factors are the following: TNF-α, IL-1, IL-6 alone or in various combinations and optionally containing prostaglandins (monocyte conditioned medium: Thurner B. et al., J. Immunol. Methods 223(1):1-15 (1999); defined cocktail: Jonuleit H. et al., Eur. J. Immunol. 27(12):3135-42 (1997)), various TLR ligands (Napolitani G. et al., Nat. Immunol. 6(8):769-76 (2005); Gautier G. et al., J. Exp. Med. 201(9):1435-46 (2005)); CD40 crosslinking by CD40 ligand and optionally containing IFN-γ (Caux C. et al., J. Exp. Med. 180(4):1263-72 (1994); Koya R.C. et al., J. Immunother. 26(5):451-60 (2003)), type I interferon (Gautier G. et al., J. Exp. Med. 201(9):1435-46 (2005)) and combinations of the above maturation factors. Particularly preferred is a maturation cocktail comprising TNF-α, IL-1β, IL-6, and PGE₂.

Before, during or after the maturation the dendritic cells the dendritic cells or the respective dendritic cell precursors may be loaded with protein or lipid antigens, DNA or RNA coding for antigens, whole cells (preferably apoptotic or necrotic cells), cell fragments or cell lysates, or the like.

The method of the invention may further include one or more of the following steps: partitioning and/or cryopreservation of the cells cultured as defined hereinbefore.

As set forth above, the method of the invention corresponds to the following solution: The problem was to perform the elutriation in a culture medium which would be suitable for both the elutriation step and the subsequent culture of the desired cells. It was found that particular media such as the RPMI 1640 + 1% autologous plasma fulfilled the two requirements, and in addition is to the best of our knowledge the cheapest of the culture media approved for clinical use (see Example). It is important to note that this solution to the problem was not evident from prior art as elutriation (as well as magnetic separation) has generally been performed in salt solutions because of the possible interference of media components. The performance of elutriation - or, more generally spoken the isolation of cells - in media close to or identical with the final culture media, appears to be a small improvement but is in fact a big one from a practical point of view. By circumventing any centrifugation step after the monocyte enrichment and the initiation of culture a very simple functionally-closed cell separation system has become possible. In case of large scale DC production it merely requires three simple steps (which require only readily available instruments and reagents approved for clinical use), namely
i) apheresis resulting in a bag/container containing mononuclear cells,
ii) elutriation (or magnetic separation or other enrichment procedures) performed in media identical or close to the final culture media to yield the desired cells in a bag/container which contains the (final or next to final) culture medium, and
iii) injection into the bag(s)/container(s) of supplements (such as cytokines like GM-CSF and IL-4)
to yield the desired final progeny (such as DC) upon culture of the cells enriched by elutriation (or magnetic separation).

The invention is further described by the following examples, which are, however, not to be construed as limiting the invention.

### Examples

### Example 1: Isolation of MNC from healthy donors.

The leukapheresis was performed with healthy donors after informed consent was given as described in Strasser, E. F. et al., Transfusion 43(9): 1309 (2003). A modified MNC program was used for the Cobe Spectra (MNC program version 5.1) with an enhanced separation factor of 700. The inlet blood flow rate was 60 ml/min with a continuous collection rate of 0.8 ml/min. The anticoagulant was set to an ACD-A to blood ratio of 1:10 as basic adjustment for all procedures that was adjusted to a ratio of 1:11 or 1:12 in case of citrate reactions. The separation factor of 700 was equivalent to a centrifugation speed of 1184 rpm at a blood flow rate of 60 ml/min. The rate of the plasma pump was set manually by visual inspection of the cell interface within the separation chamber and the observation of the collection line to target a low hematocrit of approximately 1% to 2%.

### Example 2: Enrichment of monocytes by elutriation with culture medium.

Peripheral blood monocytes obtained in Example 1 were enriched directly from immobilized leukapheresis products using an Elutra^{™} cell separator (Gambro BCT, Lakewood, Colorado, USA) and single-use, functionally sealed disposable sets, containing a newly designed 40-ml elutriation chamber. It separates cells on the basis of sedimentation velocity, which is dependent on cell size and, to a lesser extent density. After prime, the leukapheresis product was dissolved in elutriation/culture medium (glutamine-free RPMI 1640 medium BE12-167F (Bio Whittaker, Walkersville, USA) supplemented with 20 µg/ml gentamicin (Refobacin 10, Merck, Darmstadt, Germany), 2 mM glutamine (Bio Whit-taker), and 1% heat-inactivated (56 °C for 30 min) autologous human plasma) and was then loaded into the elutriation chamber using the cell inlet pump and a centrifuge speed of 2400 rpm. Thereafter, the centrifuge speed was held constant, and the flow of elutriation/culture medium contained in two 3-I pooling bags (T3006, Cell-Max GmbH, Munich, Germany), was increased step-wise to allow for the elutriation of the specific cell fractions into the pre-attached collection bags (Fig. 1). The cell inlet pump speed was set at 37 ml/min, the media pump speed was gradually increased in 4 steps, namely 37-97.5-103.4-103.9 ml/min, and ~975 ml elutriation media per fraction was collected. To collect the final fraction from the elutriation chamber (rotor off fraction), the centrifuge was stopped and the cells were pumped at 103.9 ml/min into the final collection bag. The total processing time was approximately one hour.

Cellular components of all collected fractions were manually counted by standard trypan blue dye exclusion. For performing flow cytometry, cells were labelled with anti-CD14-FITC and anti-CD45-CyCr mAbs (Becton Dickinson, New Jersey, USA) and analysed on a Cytomics FC500 (Beckman-Coulter, Miami, FL, USA) using RxP software. In addition, cells were studied in an automatic cell counter (Casy cell counter and analyzer system, model TT, Schärfe System, Reutlingen, Germany). This system uses pulse area analysis and allows for the objective measurement of cell numbers, cell size, and volume, as well as cell viability.

### Example 3: DC-generation

Monocytes obtained in Example 2 were separated into four culture bags CF100-C3 Cell-Freeze (Cell-Max GmbH, Munich, Germany; which is applicable for cryopreservation and contain tubings that allow for sterile docking) at a density of 1x10⁶/ml in 50-70 ml culture medium. under sterile conditions and were incubated at 37 °C and 5% CO₂. The bags were supplemented on days 1, 3, and 5 with rhu GM-CSF (final concentration 800 U/ml; Leukomax, Novartis, Nuremberg, Germany) and rhu IL-4 (final concentration 250 U/ml; Strathmann Biotec, Hamburg, Germany). On day 6, a maturation cocktail consisting of TNF-α (10 ng/ml, Boehringer Ingelheim Austria, Vienna, Austria) +IL-1β (10 ng/ml; Cell Concepts, Umkirch, Germany) +IL-6 (1000 U/ml; Strathmann Biotec, Hamburg, Germany) +PGE₂ (1 Ag/ml; MinprostinR, Pharmacia and Upjohn, Erlangen, Germany) (Jonuleit, H. et al., Eur. J. Immunol.27:3135 (1997)). After 24 h the cells were harvested and cryoconserved in the culture bags.

### Example 4: Cell morphology and phenotype

The morphology of DC was evaluated under an inverted phase contrast microscope (Leica DM IRB, Leica Mikroskopie und Systeme GmbH, Wetzlar, Germany) and was photographically documented. Cells were phenotyped using a panel of mAbs and analyzed on a FACScanR with cell questR software (Becton Dickinson) as described previously in more detail (Romani, N. et al., J. Immunol. Methods 196(2):137 (1996)).

### Example 5: Leukapheresis of MNC from healthy donors and patients

Leukapheresis was performed as described with minor modifications at the Department of Transfusion Medicine from healthy donors (n=5) and patients with metastasized melanoma receiving DC-vaccination therapy (n=14) after informed consent was obtained (Strasser et al., 2003). We used a modified MNC program for the Cobe Spectra (MNC program version 5.1) with an enhanced separation factor (SF) of 250. The inlet blood flow rate was 50 ml per minute with a continuous collection rate (CR) of 1.0 ml per minute. The anticoagulant was set to an ACD-A to blood ratio of 1 in 10 as basic adjustment for all procedures that was adjusted to a ratio of 1:11 or 1:12 in case of citrate reactions. The separation factor (SF) of 250 was equivalent to a centrifugation speed of 646 rpm at a blood flow rate of 50 ml per minute. The rate of the plasma pump was set manually by visual inspection of the cell interface within in the separation chamber and the observation of the collection line to target a low erythrocyte count of approximately 0,40 x 10⁶/µl.

Five research and fourteen patient leukaphereses for counterflow elutriation were performed as described above for later enrichment of monocytes by counterflow elutriation with RPMI-Medium. Key data of the leukaphereses are summarized in Table 1a. As a comparison four leukaphereses were performed for later enrichment of monocytes by counterflow elutriation with CellGro-Medium (Table 1b).

**Table 1: Parameters of leukaphereses**

| a) RPMI-Medium | | | | | |
|---|---|---|---|---|---|
| Leukapheresis | Volume [ml] | WBC [10³/µl] | Mo [%] | total Mo[10⁶] | RBC [10⁶/µl] |
| research (n=5) | 127.4 ± 32.6 | 60.4 ± 11.9 | 16.4 ± 3.7 | 1257 ± 469 | 0.33 ± 0.06 |
| Patients (n= 14) | 192.0 ± 24.2 | 56.2 ± 15.5 | 23.5 ± 4.8 | 2556 ± 961 | 0.36 ± 0.12 |

| b) CellGro-Medium | | | | | |
|---|---|---|---|---|---|
| Leukapheresis | Volume [ml] | WBC [10³/µl] | Mo [%] | total Mo[10⁶] | RBC [10⁶/µl] |
| Leu 142 | 112 | 53 | 15.18 | 977 | 0.59 |
| Leu 143 | 117 | 44.6 | 22.19 | 1197 | 0.49 |
| Leu 144 | 104 | 75.1 | 25.4 | 1983,8 | 0.37 |
| Leu 145 | 130 | 50 | 16.52 | 1073 | 0.38 |
| Arithmetic mean + Standard deviation | 115.8 ± 7.8 | 55.7 ± 9.7 | 19.8 ± 4.0 | 1307 ± 338 | 0.46 ± 0.08 |

Research leukaphereses required approximately two hours and yielded 127.4 ± 32.6 ml compared to patient leukaphereses which produced 192 ± 24.2 ml in approximately four hours. Due to the larger volume patient leukaphereses yielded 2556 ± 961 x 10⁶ monocytes compared to 1257 ± 469 x 10⁶ monocytes in research leukaphereses (Table 1a). Only minor fluctuations in WBC-, RBC-concentration and percentage of monocytes/WBC were observed between the patient and research leukaphereses as well as previous patient leukaphereses in previous years.

### Example 6 : Enrichment of monocytes by counterflow elutriation

Peripheral blood monocytes were enriched from the leukapheresis products obtained as described in example 5 by counterflow elutriation using an Elutra^{™}-cell separator (Gambro BCT, Inc. Lakewood, Colorado, USA) and single-use, functionally sealed disposable sets as described before (Berger et al., 2005).

In counterflow elutriation separation of different leukocyte population is achieved on the basis of sedimentation velocity (depending on cell-size) and to a lesser extent on density.

After priming the system with elutriation medium, the leukapheresis bag was sterilely attached and cells were loaded into the elutriation chamber at a centrifuge speed of 2400 rpm. By constant centrifuge speed the flow of elutriation medium (RPMI 1640; Bio Whittaker, Walkersville, USA, adding 1% autologous heat-inactivated plasma or CellGro-Medium) increased step-wise by the predefined settings to separate five specific fractions into the pre-attached collection bags with the final fraction 5 containing the majority of monocytes and low other PBMC-contamination. At an increasing medium pump speed of 37-97.5-103.4-103.9 ml/min approximately 900 ml elutriation medium per fraction was collected. The accumulated monocytes in the 40 ml chamber were gathered at 0 rpm and at a medium flow rate of 103.9 ml/min. Total elutriation time was one hour. Cells of the different fractions were manually counted by standard trypanblue dye exclusion and additionally by an automatic cell counter (Casy cell counter and analyzer system, model TTC, Schaerfe System, Reutlingen, Germany). The percentage of monocytes in each fraction was determined by flow cytometry (FACSCalibur 4CA and Cellquest software, BD Biosciences, San Jose, USA). For that purpose the cell populations were gated in a forward/sideward scatter and labeled with anti-CD14-FITC and anti-CD45-CyCr mab (Becton Dickinson, New Jersey, U.S.A.).

### Example 7: Monocyte-yield and -purity by Elutra^{™}

During counterflow elutriation as described in example 6 cell subsets of the leukapheresis are separated into different bags (fraction 1: mainly platelets with erythrocytes, fraction 2: mainly erythrocytes and few lymphocytes, fraction 3: WBC (mainly lymphocytes), fraction 4: WBC (mainly granulocytes) with some monocytes and in the rotor off, fraction 5: the majority of monocytes with little lymphocyte and granulocyte contamination).

The percentages of harvested monocytes in fraction 5 compared to total monocytes in the leukapheresis (F5-yield) ranged between 52.9 ± 8.3% in research and 86.7 ± 7.5% in patient leukaphereses with RPMI-Medium (Table 2a). CD14 positivity of fraction 5 cells (F5-purity) was 76.1 ± 6.8% in research and respectively 79.3 ± 6.8% in patient leukaphereses with RPMI-Medium. Due to the larger collection volume in patient leukaphereses more monocytes were collected (2289 ± 951 x 10⁶) compared to the research setting (695 ± 326 x 10⁶). The use of EC medium during the elutriation process did not alter the separation of the blood cell fractions.

**Table 2: Elutriation data (F5-yield: percentage of total leukapheresis monocytes in fraction 5, F5-purity: % of CD14+ monocytes of cells in fraction 5)**

| a) RPMI-Medium | | | |
|---|---|---|---|
| Leukapheresis | F5-yield [%] | F5-purity [%] | Mo [10⁶] |
| research (n=5) | 52.9 ± 8.3 | 76.1 ± 6.8 | 695 ± 326 |
| patient (n=14) | 86.7 ± 7.5 | 79.3 ± 6.8 | 2289 ± 951 |

| b) CellGro-Medium | | | |
|---|---|---|---|
| Leukapheresis | F5-yield [%] | F5-purity [%] | Mo [10⁶] |
| Leu 142 | 64.7 | 77.26 | 632 |
| Leu 143 | 86.9 | 92 | 1039.6 |
| Leu 144 | 66.5 | 90 | 1318.5 |
| Leu 145 | 54.6 | 60 | 586 |
| Arithmetic mean + Standard deviation | 68.2 ± 9.4 | 79.8 ± 11.2 | 894 ± 285 |

### Example 8: Differentiation of monocytes into DC

The high purity monocyte fraction 5 enriched by elutriation (as described in example 6) was diluted with EC medium (510.1 ml of EC medium consisted of 500 ml RPMI 1640 without glutamine (BioWhittaker/Cambrex, USA), 5 ml L-Glutamine (BioWhittaker/Cambrex, USA), 10 mg Refobacin (Merck, USA) and 5.1 ml heat inactivated (30 minutes at 56°C), autologous plasma (Thurner et al., 1999) or CellGro-Medium) to a density of 1,6 x 10⁶/ml and redistributed into culture bags (Cell-Freeze^{™}, Cell-Max GmbH, Munich, Germany) with a maximum of 400 ml per 2000 ml-bag and 50 ml per 100 ml-bag. IL-4 (Cell Genix, Freiburg, Germany) and GM-CSF (Leukine^{®}, Berlex, Richmond, USA) were added to final concentrations of 250 U/ml and 800 U/ml respectively.

On day 3 and 5 cells were again fed with EC medium (70 ml for 2000 ml-bags; 10 ml for 100 ml-bags) supplemented with GM-CSF and IL-4 (250 U/ml and 800 U/ml respectively). On day 6 cells were matured by MCM-mimic, consisting of TNF-α (10 ng/ml, Beromun^{®}, Boehringer Ingelheim, Ingelheim, Germany), IL-1β(Cell Genix, Freiburg, Germany), IL-6 (1000 U/ml; Cell Genix, Freiburg, Germany) and PGE₂ (1 µg/ml; Prostin E2^{®}, Pfizer, Puurs, Belgium) (Jonuleit et al., 1997). On day 7 cells were harvested and their morphology and phenotype were analyzed.

DC generated according to our adherence protocol served as controls (Thurner et al., 1999; Berger et al., 2002). For that purpose PBMC, generated via density gradient centrifugation, were plated in cell factories^{™} at a density of 5 x 10⁶ cells/ml of complete medium without cytokines and incubated at 37°C and 5% CO₂ for 1 h. Non-adherent cells were removed after one hour. The cell factories^{™} were washed with RPMI 1640 twice, and 240 ml of warm complete medium without cytokines were added (day 0). Cytokines and culture medium were added in the same way as for cultivation in culture bags, except that the first addition of cytokines was carried out on the day after plastic adherence versus immediate addition of cytokines to elutriated cells.

DC yield was calculated as the percentage of DC harvested on day 7 divided by monocytes provided by elutriation or density gradient centrifugation on day 0.

DC were counted using standard trypanblue dye exclusion and additionally a Casy cell counter (Casy cell counter and analyzer system, model TTC, Schaerfe System, Reutlingen, Germany).

In addition viability of produced DC was routinely tested by further culture in EC medium without GM-CSF and IL-4 for one day ("washout test")(Romani et al., 1996).

**Table 3: DC data on day 7 of research, patient and CD40L-studv leukaphereses (DC-yield: percentage of DC harvested compared to monocytes placed into culture; washout: percentage of viable DC after 24 h in culture)**

| a) RPMI-Medium | | |
|---|---|---|
| Leukapheresis | DC-yield [%] | washout [%] |
| research (n=5) | 31.6 ± 11.9 | 74.6 ± 9.5 |
| patient (n=14) | 47.6 ± 12.6 | 83.5 ± 10.5 |
| CD40L (n=75) | 20.1 ± 6.9 | 83.8 ± 9.7 |

| b) CellGro-Medium | | |
|---|---|---|
| Leukapheresis | DC-yield [%] | washout [%] |
| Leu 142 | 76 | 68 |
| Leu 143 | 44 | 80 |
| Leu 144 | 56 | 86 |
| Leu 145 | 54 | 100 |
| Arithmetic mean + Standard deviation | 57.5 ± 9.3 | 83.5 ± 9.5 |

### Example 9: DC morphology and phenotype

DC morphology was evaluated by inverted phase contrast microscopy (Leica DM IRB, Leica Mikroskopie und Systeme GmbH, Wetzlar, Germany) and photographically documented (Nikon Coolpix 4500, Nikon, Japan).

Phenotype and maturation of DC was determined by flow cytometry (FACSCalibur 4CA and Cellquest software, BD Biosciences, San Jose, USA). Therefore we stained with mab against HLA-DR, CD1a, CD14, CD25, CD83, and CD86 (mab from Becton Dickinson, San Jose, USA). Contaminating NK-cells, T cells and B cells were determined by CD56, CD3 and CD19-mab (mab from Becton Dickinson, San Jose, USA).

**Table 4: Phenotype of DC on day 7**

| a) RPMI-Medium | | | |
|---|---|---|---|
| Leukapheresis | CD14 % | CD83 % | HLA-DR % |
| research (n=5) | 4.6 ± 2.7 | 92.7 ± 3.5 | 77.2 ± 21.3 |
| patient (n=14) | 3.1 ± 1.9 | 88.8 ± 7.2 | 94.5 ± 5.2 |

| b) RPMI-Medium | | | |
|---|---|---|---|
| Leukapheresis | CD86 % | CD25 % | CD1a % |
| research (n=5) | 98.9 ± 0.6 | 64.9 ±13.7 | 70.9 ± 13.1 |
| patient (n=14) | 98.3 ± 1.6 | 89.5 ± 7.5 | 74.0 ± 17.9 |

| c) CellGro-Medium | | | |
|---|---|---|---|
| Leukapheresis | CD14 % | CD83 % | HLA-DR % |
| Leu 142 | 1.22 | 90.57 | 94.66 |
| Leu 143 | 1.32 | 82.59 | 98.96 |
| Leu 144 | 9.51 | 82.86 | 97.15 |
| Leu 145 | 0.09 | 80.82 | 99.74 |
| Arithmetic mean + Standard deviation | 3.0 ± 3.2 | 84.2 ± 3.2 | 97.6 ± 1.7 |

| d) CellGro-Medium | | | |
|---|---|---|---|
| Leukapheresis | CD86 % | CD25 % | CD1a % |
| Leu 142 | 97.24 | 70.55 | 40.42 |
| Leu 143 | 99.21 | 89.39 | 27.57 |
| Leu 144 | 99.74 | 94.33 | 55.16 |
| Leu 145 | 99.92 | 73.77 | 16.07 |
| Arithmetic mean + Standard deviation | 99.0 ± 0.9 | 82.0 ± 9.9 | 34.8 ± 13.0 |

**Table 5: Lymphocyte and NK cell contamination on day 7 in DC culture**

| a) RPMI-Medium | | | |
|---|---|---|---|
| Leukapheresis | CD19 % | CD56 % | CD3 % |
| research (n=5) | 0.64 ± 0.24 | 3.0 ± 1.7 | 1.2 ± 0.8 |
| patient (n=5) | 1.40 ± 0.9 | 2.3 ± 1.3 | 0.5 ± 0.5 |

| b) CellGro-Medium | | | |
|---|---|---|---|
| Leukapheresis | CD19 % | CD56 % | CD3 % |
| Leu 142 | 0.64 | 1.17 | 0.01 |
| Leu 143 | 2.09 | 0.52 | 0 |
| Leu 144 | 1.02 | 0.93 | 0.43 |
| Leu 145 | 2.51 | 2.35 | 1.09 |
| Arithmetic mean + Standard deviation | 1.6 ± 0.7 | 1.2 ± 0.6 | 0.4 ± 0.4 |

### Example 10: Allogeneic mixed lymphocyte reaction

Allogenic-MLR was performed as described (Thurner et al., 1999). Briefly, 2 x 10⁵ allogeneic non-adherent PBMC per well and DC (1:6.6; 1:20; 1:66; 1:200; 1:666; 1:2000) were co-cultured in triplicates in 96-well flat bottom plates for four days in RPMI 1640 supplemented with gentamicin, glutamine and 5 % heat-inactivated human pool serum. Proliferation of each PBMC/DC-co-cultivation was determined by ³H-Thymidine-assay (4 µCi/ml final concentration added 12-16 h before harvest). DC generated by elutriation-enriched monocytes showed adequate function in mixed lymphocyte culture (Figure 2).

### Example 11: Algorithm to circumvent debulking during counterflow elutriation

As abundant erythrocytes interfere with effectiveness in counterflow elutriation, Elutra^{™} offers a debulking step to reduce RBC before elutriation. This procedure results in a significant monocyte loss of the leukapheresis product.

Therefore we designed an algorithm to determine the maximum amount of erythrocytes which Elutra^{™} can process with a given RBC- and WBC-concentration without perfoming debulking. Elutra^{™} program performs debulking if the packed erythrocytes volume in elutriation chamber exceeds 7.5 ml. As *Packed erythrocytes volume [ml] = RBC-concentration of leukapheresis [10⁶*/*µl] x leukapheresis volume [ml] x 0.095021 (packed erythrocyte factor)* we determined the maximum volume of leukapheresis with a given RBC-concentration which resulted in a packed erythrocytes volume just below 7.5 ml (Table 6).

As counterflow elutriation requires 5 - 30 x 10⁹ WBC it is essential that the leukapheresis product contains a low RBC- and high WBC-concentration.

**Table 6: Cutoff volumes of leukaphereses for a given RBC-concentration in order to prevent debulking**

| RBC [10⁶/µl] | Volume (ml) | RBC [10⁶/µl] | Volume (ml) |
|---|---|---|---|
| 0.6 | 131.55 | 0.4 | 197.32 |
| 0.58 | 136.09 | 0.38 | 207.71 |
| 0.56 | 140.95 | 0.36 | 219.25 |
| 0.54 | 146.17 | 0.34 | 232.15 |
| 0.52 | 151.79 | 0.32 | 246.66 |
| 0.5 | 157.86 | 0.3 | 263.10 |
| 0.48 | 164.44 | 0.28 | 281.89 |
| 0.46 | 171.59 | 0.26 | 303.58 |
| 0.44 | 179.39 | 0.24 | 328.87 |
| 0.42 | 187.93 | 0.22 | 358.77 |

## Claims

1. A method for the sterile separation and culture of blood-derived cells or bone marrow-derived cells, which method comprises enriching the cells by a sterile separation procedure and performing the enrichment or elution step of the separation procedure with an eluation medium close to or identical with the medium required for culture of the cells, and collecting the cells in a vessel under sterile conditions.

2. The method of claim 1, wherein the cells are subsequently cultured in the eluation medium, preferably without a centrifugation step between the separation procedure and the culturing of the cells.

3. The method of claim 1 or 2, wherein
(i) the separation procedure is elutriation, magnetic separation etc.; and/or
(ii) the blood-derived cells or bone marrow-derived cells are selected from monocytes, immature dendritic cells, CD34⁺ cells, stem cells, and any other cell contained therein; and/or
(iii) the vessel allows for sterile collection and culture of the cell suspension, sterile transfer to other culture or storage vessels, and optionally for sterile cryopreservation, preferably the vessel is a plastic bag having sealable adapters, most preferably the vessel is a Cell-Freeze bag type CF100-C3 containing a pre-attached DMSO filter which allows for closed system cryopreservation, and/or
(iv) the method further comprises culture, differentiation and/or expansion of the cells under reaction conditions and by addition of supplements required for the culture, differentiation and/or expansion of the respective cells or precursor cells.

4. The method of any one of claims 1 to 3, which is suitable for the sterile culture or generation of dendritic cells and comprises elutriation of peripheral blood mononuclear cells and culture of the monocytes obtained by the elutriation.

5. The method of claim 4, wherein the buffer for elutriation is selected from RPMI medium supplemented with autologous plasma or serum or allogenic plasma or serum (preferably with about 1% of said plasma or serum), X-Vivo 15 medium, CellGro DC Medium, AimV Culture Medium, Panserin^{™} 416, and the like, and preferably is RPMI medium supplemented with 1% autologous human plasma or serum.

6. The method of claim 4 or 5, wherein the culture of the monocytes further comprises adding one or more differentiation factors and/or one or more maturation factors, preferably
(i) for generating stimulatory dendritic cells the differentiation factors are selected from GM-CSF, IL-4 IL-13, IFN-α, IL-3, IFN-β, TNF-α, CPG oligonucleotides and combinations of said differentiation factors, a combination of GM-CSF and IL-4, being particularly preferred; and/or
(ii) the maturation factors are selected from TNF-α, IL-1 such as IL-1β, IL-6, prostaglandins such as PGE₂, TLR ligands, CD40 crosslinking by CD40 ligand, type I interferon and combinations thereof, a maturation cocktail comprising TNF-α, IL-1β, IL-6 and PGE₂ being particularly preferred.

7. The method of any one of claims 1 to 6, which further comprises
(i) partitioning and/or cryopreservation of the cultured cells, and/or
(ii) loading the dendritic cells or the respective dendritic cell precursors with protein or lipid antigens, DNA or RNA coding for antigens, whole cells (preferably apoptotic or necrotic cells), cell fragments or cell lysates.

8. A method according to any one of claims 1 to 7, wherein an elutriation device, preferably the Elutra^{™} Cell Separation System is utilized in the separation procedure.

9. A method according to claim 8, wherein the elutriation device is operated so to avoid debulking of erythrocytes during elutriation in order to prevent monocyte loss of the leukapheresis product.

10. A method according to claim 9, wherein debulking of erythrocytes is prevented by limiting the input volume of leukapheresis product to the elutriation device relative to the erythrocyte concentration, preferably wherein debulking is prevented by limiting the input volume of leukapheresis product to the elutriation device so that the packed erythrocyte volume is less than 20% of the volume of the reaction chamber of the elutriation device.

11. The method of any one of claims 1 to 10, wherein the cells, in particular the dendritic cells obtained by the process are suitable to be transfused or retransfused to a patient.
